# EUROPÄISCHE PATENTSCHRIFT

(11) **EP 2 393 529 B1**
(45) Veröffentlichungstag und Bekanntmachung des Hinweises auf die Patenterteilung: **23.10.2019**
(21) Anmeldenummer: 10703811.9
(22) Anmeldetag: 05.02.2010
(51) Int. Cl.: A61M 1/36, A61M 5/168, A61M 5/158

(54) **VORRICHTUNG ZUR SCHWINGUNGSANREGUNG WENIGSTENS EINES ABSCHNITTS EINER GEFÄSSZUGANGSEINRICHTUNG ZU DEREN ÜBERWACHUNG**
DEVICE FOR EXCITING VIBRATION OF AT LEAST ONE SEGMENT OF A VASCULAR ACCESS DEVICE FOR MONITORING THE SAME
SYSTÈME POUR SOUMETTRE AU MOINS UNE PARTIE D'UN DISPOSITIF D'ACCÈS VASCULAIRE À DES VIBRATIONS À DES FINS DE CONTRÔLE

(30) Priorität: 06.02.2009 DE 102009007806
(43) Veröffentlichungstag der Anmeldung: 14.12.2011
(73) Patentinhaber: Fresenius Medical Care Deutschland GmbH, 61352 Bad Homburg (DE)
(72) Erfinder: THYS, Martin, 97508 Grettstadt (DE)
(74) Vertreter: Bobbert & Partner Patentanwälte PartmbB
(86) Internationale Anmeldenummer: PCT/EP2010/000724
(87) Internationale Veröffentlichungsnummer: WO 2010/089130

(56) Entgegenhaltungen:
- WO-A1-97/10013
- WO-A1-2009/038834
- WO-A1-2009/122229
- US-A1- 2008 195 021

## Beschreibung

Die vorliegende Erfindung betrifft eine Vorrichtung zur Schwingungsanregung wenigstens eines Abschnitts einer Gefäßzugangseinrichtung gemäß dem Oberbegriff des Anspruchs 1.

Verschiedene Behandlungsverfahren erfordern den Einsatz einer mit dem Gefäßsystem eines Patienten konnektierbaren Gefäßzugangseinrichtung. Dabei erfolgt die Konnektion üblicherweise durch Einführen von wenigstens einem Abschnitt der Gefäßzugangseinrichtung in das Gefäßsystem des Patienten. Während des Behandlungsverfahrens wird die Verbindung oder Konnektion durch Fixieren von wenigstens einem Teil der Gefäßzugangseinrichtung sichergestellt.

Verschiedene Ursachen, wie beispielsweise Bewegungen des Patienten, können trotz getroffener Sicherheitsmaßnahmen wie dem zuvor genannten Fixieren zu einer unerwünschten Dekonnektion der Gefäßzugangseinrichtung vom Gefäßsystem des Patienten führen. Eine solche Dekonnektion kann mit schwerwiegenden Konsequenzen für den Patienten wegen Freifluss des Bluts verbunden sein und sollte daher möglichst verhindert oder zumindest so schnell wie möglich erkannt und behoben werden.

Aus dem Stand der Technik sind verschiedene Vorrichtungen und Verfahren zum Erkennen einer solchen Dekonnektion bekannt.

So ist aus der Praxis beispielsweise bekannt, medizinische Geräte mittels Körperschallmessung zu überwachen, und Änderungen in der Ausbreitung von Schall im Gerät (wie einem Blutschlauchsystem) oder im Körper des Patienten zum Feststellen einer Dekonnektion der Gefäßzugangseinrichtung vom Gefäßsystem des Patienten heranzuziehen.

Weitere aus der Praxis bekannte Verfahren schließen das Erkennen von Feuchtigkeit an der Konnektionsstelle ein, wie durch ein Austreten von Blut bewirkt.

Aus der US 2008/195021 A1 sind akustische Zugangstrennungssysteme und -verfahren bekannt.

Aus der WO 97/10013 A1 sind eine Methode und eine Vorrichtung zur Aufzeichnung des Zustandes eines Blutgefäßzugangs bekannt.

In der WO 2099/038834 A ist ein akustisches Zugangsunterbrechungsdetektionssystem offenbart.

Aus der WO 2099/122229 A1 sind eine Vorrichtung und ein Verfahren zur Überwachung eines Gefäßzugangs bekannt.

Es ist Aufgabe der vorliegenden Erfindung, eine wiederum weitere Vorrichtung zum Überwachen einer Gefäßzugangseinrichtung bereitzustellen.

Die erfindungsgemäße Aufgabe wird durch eine Vorrichtung mit den Merkmalen des Anspruchs 1 gelöst.

Die erfindungsgemäße Vorrichtung ist zur Schwingungsanregung wenigstens eines Abschnitts einer Gefäßzugangseinrichtung oder der gesamten Gefäßzugangseinrichtung zum Überwachen der Gefäßzugangseinrichtung geeignet und vorgesehen.

Zu diesem Zweck umfasst die erfindungsgemäße Vorrichtung wenigstens einen Signalgeber zum Erzeugen von Schwingungen zum Anregen des Abschnitts der Gefäßzugangseinrichtung zum Schwingen, wobei der Abschnitt der Gefäßzugangseinrichtung dem Herstellen einer Verbindung zwischen der Gefäßzugangseinrichtung und dem Gefäßsystem des Patienten dient und wenigstens zum Teil in das Gefäßsystem einführbar ist. Die erfindungsgemäße Vorrichtung weist ferner wenigstens einen Signalempfänger zum Erfassen von Schwingungen des Abschnitts der Gefäßzugangseinrichtung und wenigstens eine Auswerteeinrichtung zum Auswerten der erfassten Schwingungen auf.

Der Signalgeber und/oder der Signalempfänger können Schallgeber und -empfänger sein. Die Schwingungen können bevorzugt als Schallwellen zu verstehen sein.

Der Begriff "Anregen zum Schwingen" oder "Schwingungsanregung", wie er vorliegend verwendet wird, bezeichnet einen Vorgang, bei dem eine Einrichtung oder wenigstens ein Abschnitt derselben ausgehend von einem ersten Schwingungszustand derart in Schwingung versetzt wird oder werden soll, dass er in einen zweiten Schwingungszustand, sich vom ersten regelmäßig unterscheidenden Schwingungszustand, übergeht.

Dabei kann es sich bei dem ersten Schwingungszustand um einen Ruhezustand, also ein Nichtschwingen, oder bereits um einen schwingenden Ausgangszustand, beispielsweise mit einer gegebenen Frequenz und/oder Amplitude, handeln. Der zweite Schwingungszustand, welcher nach dem Anregen erreicht wird, kann ein Zustand des Schwingens mit höherer Frequenz und/oder größerer Amplitude als jene des ersten Schwingungszustands sein.

Ferner kann ein solches Anregen bzw. eine solche Schwingungsanregung auch eine Schwingungsberuhigung einer bereits schwingenden Einrichtung sein, also von einem ersten Schwingungszustand zu einem zweiten Schwingungszustand führen, wobei sich der zweite Schwingungszustand durch ein Schwingen mit geringerer Frequenz und/oder kleinerer Amplitude oder durch ein Nichtschwingen auszeichnen kann.

Ein Anregen kann erfindungsgemäß somit jeder Versuch einer - insbesondere gezielten - Beeinflussung oder eines - wiederum insbesondere gezielten - Veränderns eines Schwingungszustands sein.

Eine "Gefäßzugangseinrichtung", wie sie vorliegend bzw. hier verwendet wird, dient zum Herstellen einer Fluidverbindung zwischen einem Äußeren und einem Inneren eines beliebigen Gefäßes oder einer anderen Struktur, in welcher ein Fluid dauerhaft oder vorübergehend vorliegt. Insbesondere kann sie vorzugsweise zum Herstellen einer Fluidverbindung zwischen einem Äußeren und einem Inneren des Gefäßsystems eines Patienten dienen. Dabei kann ein "Patient" im Sinne der vorliegenden Erfindung sowohl ein Mensch als auch ein Tier sein. Keinesfalls ist die vorliegende Erfindung jedoch auf ihre Anwendung zum Überwachen einer Gefäßzugangseinrichtung zum Herstellen einer Fluidverbindung mit dem Gefäßsystem eines Patienten beschränkt.

Eine solche "Fluidverbindung" kann dazu eingesetzt werden Fluide, und vorzugsweise insbesondere Körperflüssigkeiten wie Blut, aus dem Gefäß oder dem Gefäßsystem eines Patienten abzuführen und/oder Fluide in das Gefäß oder das Gefäßsystem des Patienten einzubringen.

Mittels einer Fluidverbindung können ferner Körperflüssigkeiten, z. B. nach einer Behandlung und/oder Aufreinigung derselben in einem extrakorporalen Kreislauf, in das Gefäßsystem des Patienten rückgeführt werden.

Obwohl es im Wesentlichen genügen kann, mittels des Signalgebers bevorzugt nur einen Abschnitt der Gefäßzugangseinrichtung zu Schwingungen anzuregen, kann natürlich auch die gesamte Gefäßzugangseinrichtung einen angeregten Zustand erreichen. Im Folgenden werden die Begriffe "Gefäßzugangseinrichtung" und "Abschnitt der Gefäßzugangseinrichtung" daher der Einfachheit halber gleichbedeutend für die zu Schwingungen angeregte oder anzuregende Komponente verwendet.

Dabei können sowohl eine kurzzeitige als auch eine längerfristige Konnektion zwischen der Gefäßzugangseinrichtung und dem Gefäßsystem in Betracht kommen. Die erfindungsgemäße Vorrichtung ist daher sowohl für beispielsweise Verweilkatheter als auch für deutlich kürzer liegende Zugänge, beispielsweise zur Applikation einer Antibiose einsetzbar.

Ein von der erfindungsgemäßen Vorrichtung umfasster "Signalgeber" kann, ohne darauf beschränkt zu sein, ein piezoelektrischer oder elektrodynamischer Signalgeber sein.

Dieser kann, wiederum ohne hierauf beschränkt zu sein, akustische Schwingungen zum Anregen eines Abschnitts der Gefäßzugangseinrichtung erzeugen.

Der Signalgeber kann in räumlicher Nähe der Gefäßzugangseinrichtung angeordnet sein.

Das vom Signalgeber ausgegebene Signal kann direkt, d. h. ohne Verstärkung und/oder ohne Modulation oder dergleichen zur Schwingungsanregung der Gefäßzugangseinrichtung eingesetzt werden.

Ferner ist es auch möglich, das vom Signalgeber ausgegebene Signal mit Hilfe weiterer geeigneter Einrichtungen über eine räumliche Distanz zu der Gefäßzugangseinrichtung zu übertragen bzw. vom Signalgeber erzeugte Energieformen in Schwingungen zur Anregung der Gefäßzugangseinrichtung umzuwandeln.

Ein Signalempfänger der vorliegenden Vorrichtung kann ein von der Gefäßzugangseinrichtung rückübertragenes und/oder reflektiertes Signal detektieren oder erfassen.

Das Signal kann ein Schallsignal sein. Das Signal kann eine Schwingung beschreiben oder durch eine solche verkörpert sein.

Die erfindungsgemäße Vorrichtung kann weitere für derartige wie die oben genannten Zwecke geeignete Einrichtungen aufweisen.

Obwohl die vorliegende Erfindung an verschiedenen Stellen auf Schallsignale zur Schwingungsanregung Bezug nimmt, ist die vorliegende Erfindung keinesfalls auf die alleinige Verwendung von Schallsignalen beschränkt.

Ebenso können Kombinationen von Schallsignalen mit Signalen anderer Energieformen, wie beispielsweise Temperatur, Druck, Strom und jeder weiteren für die Zwecke der vorliegenden Erfindung geeigneten Energieform erfindungsgemäß in Betracht gezogen werden. Signalgeber, Signalempfänger und weitere Einrichtungen können entsprechend ausgewählt werden.

Eine "Auswerteeinrichtung", wie sie hierin verwendet wird, kann zum Auswerten der vom Signalempfänger erfassten durch Schall und/oder weitere Energieformen erzeugten Schwingungssignale verwendet werden.

Die ausgewerteten Parameter können, ohne darauf beschränkt zu sein, Frequenz und Amplitude umfassen.

Die erfindungsgemäße Vorrichtung dient zum Überwachen einer Gefäßzugangseinrichtung. Die Vorrichtung kann dabei ausgestaltet sein, eine Position derselben zu überwachen. Sie kann ausgestaltet sein, eine Füllung oder Nichtfüllung, z. B. mit einem Fluid, zu überwachen. Auch Kombinationen der zuvor genannten Parameter können in einer erfindungsgemäßen Ausführungsform überwacht werden.

Durch Auswerten der erfassten Schwingungen kann auf eine Änderung der Position der Gefäßzugangseinrichtung geschlossen werden. Eine solche Änderung kann Hinweise auf eine Störung der Konnektion zwischen der Gefäßzugangseinrichtung und dem Gefäßsystem des Patienten, wie beispielsweise eine bereits eingetretene oder drohende Dekonnektion derselben, geben.

Durch Auswerten der erfassten Schwingungen kann auf einen Füllungszustand der Gefäßzugangseinrichtung oder dessen Änderung geschlossen werden. Eine solche Änderung kann ebenfalls Hinweise auf eine Störung der Konnektion zwischen der Gefäßzugangseinrichtung und dem Gefäßsystem des Patienten geben.

Vorteilhafte Weiterentwicklungen der vorliegenden Erfindung sind jeweils Gegenstand der Unteransprüche.

Eine "Gefäßzugangseinrichtung" einer bevorzugten Ausführungsform kann beispielsweise als Kanüle, Infusionseinrichtung oder dergleichen ausgestaltet sein und einen längerfristigen oder kurzzeitigen Zugang zum Gefäßsystem des Patienten erlauben.

Entsprechende Gefäßzugangseinrichtungen können je nach Anwendung ausgewählt werden und schließen kommerziell erhältliche Einwegartikel ein.

Derartige Gefäßzugangseinrichtungen können aus Metallen, Kunststoffen, Formgedächtnislegierungen, elektrischen Isolatoren und dergleichen gebildet sein.

Die Gefäßzugangseinrichtung kann geeignet sein, um mit einem Shunt, einer Fistel, einer arteriellen oder venösen Gefäßbahn des Patienten eine Fluidverbindung herzustellen.

Ein "Abschnitt einer Gefäßzugangseinrichtung" kann z. B. eine Punktionseinrichtung, wie eine Punktionsnadel, ein Punktionsflügel, z. B. eine arterielle oder venöse Konnektionsnadel eines DoubleNeedle-Zugangs für eine extrakorporale Blutbehandlung oder die Nadel eines SingleNeedle-Zugangs oder ein Katheter oder jede weitere Einrichtung sein, die zum Herstellen einer Verbindung zwischen der Gefäßzugangseinrichtung und dem Gefäßsystem des Patienten wenigstens zum Teil in selbiges einführbar ist und so einen Zugang zum Gefäßsystem des Patienten zulässt, ferner Abschnitte, Bereiche oder Teilbereiche davon.

Unter dem "Gefäßsystem des Patienten" kann hier - wie zuvor bereits ausgeführt - der Blutkreislauf des Patienten als anatomische Struktur verstanden werden, welche arterielle und venöse Leitungsstrukturen des Körpers oder Abschnitte hiervon umfasst.

Die Gefäßzugangseinrichtung kann zur Durchführung eines Behandlungsverfahrens geeignet und vorgesehen sein. Zu "Behandlungsverfahren" im Sinne der vorliegenden Erfindung zählen alle zum Behandeln eines Patienten üblichen Verfahren, die unter Einsatz einer mit dem Gefäßsystem des Patienten konnektierbaren Gefäßzugangseinrichtung ausführbar sind oder eine solche benötigen. Ohne darauf beschränkt zu sein, können solche Verfahren extrakorporale Blutbehandlungsverfahren, wie Hämodialyse, Hämofiltration, Hämodiafiltration aber auch Zellseparationsverfahren, Apherese, Medikationen und dergleichen, einschließen.

Unter dem Begriff "das Äußere" können alle Bereiche außerhalb des Gefäßes, insbesondere extrakorporale Bereiche oder Einrichtungen verstanden werden, die zum Aufnehmen eines aus dem Gefäß und insbesondere aus dem Gefäßsystem eines Patienten auszuführenden und/oder eines in das Gefäßsystem des Patienten ein- und/oder rückzuführenden Fluids geeignet sind. Beispiele schließen Aufbewahrungsvorrichtungen, wie Beutel mit Flüssigkeiten, insbesondere Substituatflüssigkeiten, wie isotone Kochsalzlösung, z. B. 0,9%-ige NaCl-Lösung, weitere Infusionslösungen, Abschnitte extrakorporaler Blutkreisläufe zum Behandeln von Blut des Patienten, wie z. B. Abschnitte eines extrakorporalen Schlauchsystems, Vorrichtungen zum Aufnehmen von Medikamenten zu deren Applikation und dergleichen ein.

Das Äußere kann auch die Atmosphäre bezeichnen.

Die erfindungsgemäße Vorrichtung kann geeignet sein zum Überwachen einer Konnektion, insbesondere einer venösen Konnektion, beispielsweise während eines Verfahrens zum Entfernen von Blut aus einem extrakorporalen Blutkreislauf für eine Behandlungsvorrichtung nach Beenden einer Blutbehandlungssitzung, in welcher die Gefäßzugangseinrichtung bereits vom Gefäßsystem eines Patienten dekonnektiert ist, also keine Fluidverbindung mehr besteht.

Wenn es geeignet scheint, kann das vom Signalempfänger erfasste Signal vor dessen Auswertung verstärkt, abgeschwächt, weiter- und/oder umgeleitet werden. Die Auswerteeinrichtung kann, insbesondere unmittelbar, an den Signalgeber und/oder den Signalempfänger gekoppelt und/oder körperlich mit diesem verbunden sein.

Die Auswerteeinrichtung kann ausgestaltet sein, um die Auswertung automatisch in bestimmten Zeitabständen oder kontinuierlich durchzuführen.

Die Auswerteeinrichtung kann am Patienten und/oder in oder an einer "Behandlungsvorrichtung" zum Ausführen eines Behandlungsverfahrens vorliegen und fest mit dieser integriert oder austauschbar angeordnet sein. Eine Behandlungsvorrichtung im Sinne der vorliegenden Erfindung kann eine Vorrichtung zum Ausführen eines der vorstehend genannten beispielhaften Behandlungsverfahren, wie beispielsweise eine Dialysevorrichtung, sein.

Das "Auswerten" der erfassten Schwingungen kann analog oder digital erfolgen und auf Erfahrungswerten und/oder bereits erfassten Schwingungsmustern beruhen.

Das Auswerten kann manuell gesteuert oder automatisiert erfolgen, z. B. mit Hilfe eines entsprechenden Softwareprogramms.

In einer weiteren bevorzugten Ausführungsform ist die Auswerteeinrichtung der erfindungsgemäßen Vorrichtung dazu ausgelegt, ein Schwingungsverhalten und/oder eine mechanische Dämpfung und/oder ein Ausschwingverhalten des Abschnitts der Gefäßzugangseinrichtung auszuwerten.

Ferner kann die Auswerteeinheit dazu vorbereitet und geeignet sein, eine Analyse durch Auswerten einer vom Signalsender aufgenommenen Energie durchzuführen.

Dies kann insbesondere dann erfolgen, wenn ein Schwinger bzw. ein Schwingungssystem periodisch mit niedriger Frequenz erregt und die Frequenz fortwährend erhöht wird ("Sweep"). Erreicht die Erregerfrequenz die Nähe der Eigenfrequenz(en) des Schwingers bzw. Schwingungssystems (Resonanz) und wird die Amplitude des Signalsenders begrenzt, so wird der Schwinger allgemein einen niedrigeren Betrag an Energie aufnehmen. Beispielsweise fließt bei einem elektrodynamischen Signalgeber bzw. Erreger in der Nähe der Resonanz ein geringerer Strom bei konstanter Spannungsamplitude des Signalgebers bzw. Erregers. Der Grund hierfür ist das Ansteigen der Impedanz des Signalgebers auf ein Maximum.

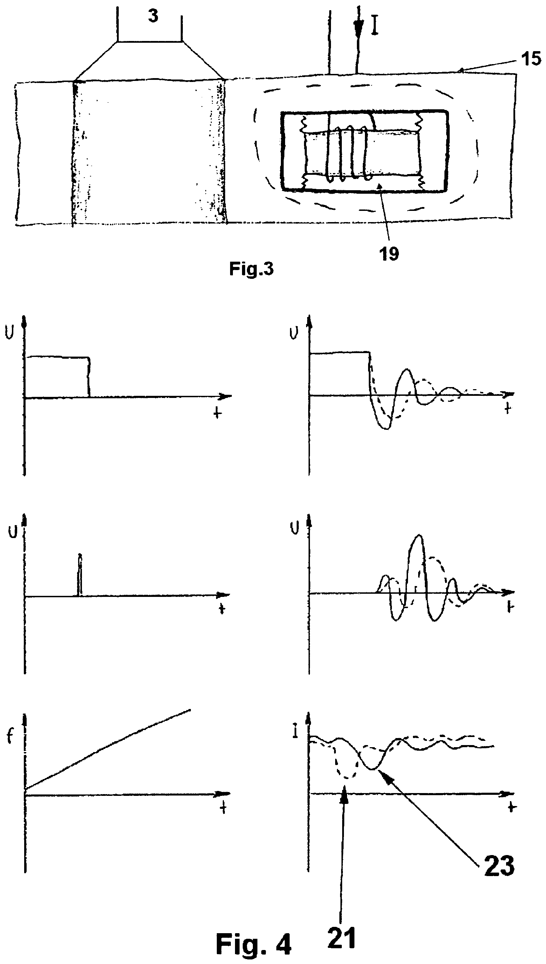

Wie nachfolgend auch unter Bezugnahme auf die Figuren und insbesondere Figur 4 der Zeichnung erläutert wird, kann eine solche Änderung der vom Signalsender aufgenommenen Energie einer Änderung des Schwingungsverhaltens entsprechen und damit Rückschlusse auf die Konnektion einer Gefäßzugangseinrichtung zulassen. Dabei kann der Effekt der "erzwungenen Schwingung" genutzt werden.

Einrichtungen zum Speichern und/oder Aufzeichnen der erfassten Schwingungssignale, eines Schwingungsverhaltens, wie beispielsweise eines Ausschwingverhaltens, und/oder bereits erfasster Schwingungsmuster können dauerhaft oder nur zu gewünschten Zeitpunkten mit der Auswerteeinrichtung oder anderen Abschnitten der erfindungsgemäßen Vorrichtung verbunden sein.

Der Begriff "Schwingungsverhalten" kann hierbei als aktuell erfasster und auszuwertender tatsächlich erfasster Schwingungszustand und/oder als Ablauf verschiedener Schwingungszustände in einem - insbesondere kurzen - Zeitintervall oder als Änderungen von Schwingungen (relativ und/oder über der Zeit) verstanden werden, während der Begriff "Schwingungsmuster" ein für ein korrektes Funktionieren erforderliches Schwingungsverhalten der Gefäßzugangseinrichtung während eines bestimmten Behandlungsverfahrens meint und als solches eine charakteristische Größe eines bestimmten Behandlungsverfahrens und/oder einer bestimmten Gefäßzugangseinrichtung darstellen kann.

In einer bevorzugten Ausführungsform der vorliegenden Erfindung sind der Signalgeber und der Signalempfänger räumlich in einer Struktur miteinander verbunden.

Der Begriff "räumlich miteinander verbunden" meint hierbei, dass der Signalgeber und der Signalempfänger auf einer gemeinsamen Trägerstruktur und/oder in einem gemeinsamen Gehäuse angeordnet sein können.

In einer Weiterbildung der vorliegenden Erfindung sind der Signalgeber und der Signalempfänger körperlich miteinander verbunden, das heißt, dass die beiden Einrichtungen durch kraft-, form- und/oder stoffschlüssige Verbindungen miteinander in Kontakt stehen.

Der Signalgeber und der Signalempfänger können zwei Einrichtungen sein oder in einer gemeinsamen vereint vorliegen.

In einer weiter bevorzugten Ausführungsform der erfindungsgemäßen Vorrichtung sind der Signalgeber und der Signalempfänger vorgesehen und geeignet, um an der Gefäßzugangseinrichtung angeordnet zu werden.

Ein solches Anordnen kann ein dauerhaftes oder lösbares Verbinden umfassen.

Ferner weist die erfindungsgemäße Vorrichtung in einer weiter bevorzugten Weiterbildung eine Befestigungseinrichtung zum Befestigen der Vorrichtung oder von Teilen hiervon an der Gefäßzugangseinrichtung auf.

Eine solche Befestigungseinrichtung kann z. B. eine Trägerstruktur und/oder ein Gehäuse und weitere Einrichtungen zum kraft- und/oder formschlüssigen Befestigen der erfindungsgemäßen Vorrichtung an der Gefäßzugangseinrichtung umfassen.

Die Befestigungseinrichtung kann ebenso Durchgangsöffnungen, Ösen und dergleichen zum dauerhaften oder lösbaren Verbinden der Befestigungseinrichtung an der Gefäßzugangseinrichtung aufweisen.

In bevorzugten Weiterbildungen kann die Befestigungseinrichtung als Clip, Klammer oder Hülse oder dergleichen ausgebildet sein. Eine Klammer kann derart ausgestaltet sein, dass sie auch nach einer Punktion eines Gefäßes an der Punktionseinrichtung, beispielsweise einer Kanüle, befestigbar ist.

Besonders geeignete Materialien hierfür können Kunststoffe, Metalle, Verbundstoffe und dergleichen umfassen, welche dazu ausgelegt sind, die Schwingungssignale und/oder deren Erzeugung, Erfassung und/oder Auswertung nicht zu beeinflussen, wie beispielsweise zu verfälschen.

Die Befestigungseinrichtung kann beliebig ausgeformt sein. Sie ist vorzugsweise ausgestaltet, um Schwingungen von dem mit ihm verbundenen Signalgeber und/oder Signalempfänger auf die Gefäßzugangseinrichtung zu übertragen oder von dieser erfassen zu lassen.

Eine weiter bevorzugte Ausführungsform schließt das leitergebundene Verbinden der Auswerteeinrichtung mit dem Signalgeber und/oder dem Signalempfänger, wie beispielsweise über ein elektrisches Kabel, ein.

Ferner kann die Auswerteeinrichtung in einer weiteren Ausführungsform in drahtloser Kommunikationsverbindung mit dem Signalgeber und/oder dem Signalempfänger stehen. Dies schließt z. B. eine Kommunikationsverbindung über Infrarot, RFID (Radio Frequency Identification; Identifizierung mit Hilfe von elektromagnetischen Wellen), Bluetooth, WLAN und dergleichen ein, wie sie für die Zwecke der vorliegenden Erfindung geeignet scheint.

Die erfindungsgemäße Vorrichtung kann die jeweils erforderlichen Einrichtungen zum Erzeugen und Sicherstellen einer derartigen Kommunikationsverbindung aufweisen.

In einer wiederum weiter bevorzugten Ausführungsform schließt die erfindungsgemäße Vorrichtung eine Einrichtung zum Be- oder Verarbeiten der empfangenen Signale ein. Eine solche Einrichtung kann beispielsweise zum Filtern, Mitteln der empfangenen Signale und/oder zur Rauschunterdrückung und dergleichen ausgelegt sein.

Eine weiter bevorzugte Ausführungsform schließt das Vorhandensein einer Einrichtung zum Ermitteln einer Dämpfung der von der Gefäßzugangseinrichtung ausgesendeten Schwingungssignale oder eines Schwingungsverhaltens derselben ein.

Ferner kann eine Dämpfung bzw. ein Dämpfungsmuster in einer Einrichtung einer weiteren bevorzugten Weiterbildung der vorliegenden Erfindung mit bekannten Dämpfungen, wie beispielsweise einem bekannten Dämpfungsmuster, mittels entsprechender Einrichtungen verglichen werden.

Die erfindungsgemäße Vorrichtung kann vorzugsweise eine Einrichtung zum Vergleichen eines ermittelten Schwingungsverhaltens bzw. eines Schwingungsmusters mit bekannten Schwingungsverhalten bzw. Schwingungsmustern aufweisen, wie in einer weiter bevorzugten Ausführungsform vorgesehen.

Das Verarbeiten der empfangenen Signale, das Ermitteln einer Dämpfung, das Vergleichen der Dämpfung mit einer bekannten Dämpfung und/oder das Vergleichen eines Schwingungsverhaltens mit einem bekannten Schwingungsverhalten kann ebenso in der Auswerteeinrichtung der erfindungsgemäßen Vorrichtung erfolgen.

Ferner können die Schwingungs- und/oder Dämpfungsmuster unter Verwendung geeigneter Speichereinrichtungen in der Auswerteeinrichtung gespeichert sein.

Eine solche Einrichtung kann z. B. in Form einer elektronischen Einrichtung, wie beispielsweise einem Chip, oder einer anderen Datenverarbeitungseinrichtung, ausgebildet sein.

Die Vorrichtung gemäß der vorliegenden Erfindung kann weitere Einrichtungen umfassen. Diese schließen beispielsweise Einrichtungen zum Ausgeben eines akustischen und/oder optischen Signals, wie beispielsweise eines Alarmsignals, Einrichtungen zum Steuern und/oder Regeln von Fluidverbindungen der Behandlungsvorrichtung basierend auf dem Ergebnis der Überwachung bzw. aufgrund der erfassten und gegebenenfalls ausgewerteten Signale und/oder der Gefäßzugangseinrichtung, wie beispielsweise Sperreinrichtungen, z. B. Klemmen, wie eine arterielle und/oder venöse Schlauchklemme einer extrakorporalen Blutbehandlungsvorrichtung, Absperrventile, Stoppeinrichtungen, z. B. Steuerungs- und/oder Regelungseinrichtungen und/oder Stoppschalter und dergleichen zum Stoppen einer Fluidfördereinrichtung, wie beispielsweise einer arteriellen Blutpumpe, und dergleichen ein.

Eine weiter bevorzugte Ausführungsform schließt eine Steuerungs- und/oder Regelungseinrichtung zum Beeinflussen der Zuführung eines Mediums über die Gefäßzugangseinrichtung ein. Eine solche Steuerung- und/oder Regelungseinrichtung kann beispielsweise mit einer vorstehend genannten Sperr- und/oder Stoppeinrichtung und entsprechenden Einrichtungen zum Ermitteln und Auswerten vorgegebener Schwellenwerte zum Zuführen bzw. Nicht-Zuführen eines Mediums verbunden sein. Das "Medium" kann Blut, eine Substituatflüssigkeit, eine Infusionslösung, Arzneimittellösungen und dergleichen umfassen.

Die Einrichtungen der erfindungsgemäßen Vorrichtung können automatisierte Einrichtungen sein und miteinander über weitere Steuerungs- und/oder Regelungseinrichtungen verbunden sein.

Eine bevorzugte Ausführungsform schließt ferner eine Kommunikationsverbindung zum Verbinden des Signalgebers und/oder des Signalempfängers mit der Auswerteeinrichtung und/oder mit einer Behandlungsvorrichtung, wie beispielsweise einer Blutbehandlungsvorrichtung, z. B. einer Dialysevorrichtung ein.

In einer bevorzugten Ausführungsform ist die Gefäßzugangseinrichtung eine Punktionseinrichtung oder eine Infusionseinrichtung.

Die erfindungsgemäße Vorrichtung kann ferner eine solche Gefäßzugangseinrichtung umfassen. Dabei kann die Gefäßzugangseinrichtung bevorzugt ein elektrisch isolierendes Material aufweisen oder aus diesem bestehen. Dabei kann es genügen, wenn nur der Abschnitt der Gefäßzugangseinrichtung, welcher zur Schwingungsanregung von Interesse ist, aus einem elektrisch isolierenden Material besteht oder ein solches aufweist, z. B. überzogen ist.

Die vorliegende Erfindung kann in vorteilhafter Weise zum einfachen und technisch und apparativ wenig aufwändigen Überwachen einer Gefäßzugangseinrichtung eingesetzt werden.

Durch Auswerten der von der Gefäßzugangseinrichtung empfangenen Schwingungssignale kann auf eine aktuelle Position derselben geschlossen werden. Damit kann in vorteilhafter Weise eine Positionsänderung oder gar ein Herausrutschen des in das Gefäßsystem des Patienten eingeführten Abschnitts der Gefäßzugangseinrichtung erkannt werden. Somit können vorbeugende Maßnahmen ergriffen werden. Wenn ein solcher Störfall bereits eingetreten ist, kann die vorliegende Erfindung ein schnelles Handeln des Behandlungspersonals erlauben und damit gegebenenfalls mögliche schwerwiegende Konsequenzen für einen Patienten verhindern.

Besonders vorteilhaft kann dies bei einer extrakorporalen Blutbehandlung sein, wo ein unbemerktes Herausrutschen einer Konnektionsnadel aus dem Gefäßsystem des Patienten einen Blutverlust oder ein Verbluten desselben bewirken kann.

Durch Vorsehen von miteinander in Kommunikationsverbindung stehenden Einrichtungen, wie Auswerteeinrichtung, Signalgebungseinrichtung, Sperr- und/oder Stoppeinrichtungen, kann ein Freifluss des Bluts des Patienten in die Umgebung vorteilhaft gestoppt werden.

Alarmgebende Einrichtungen können weiterhin zum sicheren Wahrnehmen eines Störfalls beitragen und nicht in unmittelbarer Nähe befindliches Behandlungspersonal aufmerksam machen.

Die vorliegende Vorrichtung kann in vorteilhafter Weise den Dämpfungszustand einer Gefäßzugangseinrichtung erfassen und mit bekannten Dämpfungsmustern vergleichen. Dazu muss kein Abschnitt der Gefäßzugangseinrichtung zwingend in das Gefäß und insbesondere in das Gefäßsystem des Patienten eingeführt sein. Auf diese Weise kann die vorliegende Überwachung beispielsweise unabhängig von der Anwesenheit eines Patienten durchgeführt werden. Auch in Fällen, in welchen die Gefäßzugangseinrichtung, z. B. die Nadel, nicht konnektiert ist, kann eine Überwachung derselben erfolgen.

Da unter bestimmten Umständen bereits auf einen Störfall geschlossen werden kann, wenn das anhand von bekannten Schwingungs- bzw. Dämpfungsmustern erwartete Schwingungsverhalten ausbleibt, kann z. B. auch erkannt werden, wenn beispielsweise die überwachte Gefäßzugangseinrichtung vor einer Behandlung eines Patienten noch nicht mit dem Gefäßsystem des Patienten verbunden ist. Ebenso kann es möglich sein, im dekonnektierten Zustand der Gefäßzugangseinrichtung zu erkennen, ob die Gefäßzugangseinrichtung bzw. ein Abschnitt derselben bereits mit einer Flüssigkeit gefüllt ist.

Mittels der Vorrichtung gemäß der vorliegende Erfindung kann vorteilhaft bereits auch ein unbeabsichtigtes Lösen des Signalgebers und/oder des Signalempfängers von der Gefäßzugangseinrichtung erkannt werden.

Da Befestigungseinrichtungen, wie Clip, Klammer oder Hülse, zum Aufnehmen der erfindungsgemäßen Vorrichtung lösbar an der Gefäßzugangseinrichtung befestigt werden können, können sie in vorteilhafter Weise mit Einweg-Gefäßzugangseinrichtungen wie Punktionsflügeln, Punktionsnadeln oder ganzen Blutschlauchsätzen kombiniert und selbst immer wieder verwendet werden. Letzteres trägt zum Vermeiden von Kosten für Herstellung und Logistik wie beispielsweise Transport und Lagerhaltung bei. Die bekannten Einweg-Gefäßzugangseinrichtungen können ferner vorteilhaft weiterhin in unveränderter Form verwendet werden.

Anders als bei einer Überwachung der akustischen oder elektrischen Leitfähigkeit einer "Gefäßschleife" bei zwei Gefäßzugängen - wie dies aus dem Stand der Technik bekannt ist - kann mit der vorliegenden Erfindung auch ein Fluidsystem überwacht werden, welches nur einen Gefäßzugang aufweist. Als Beispiel sei hier ein SingleNeedle-Verfahren genannt.

Ist die Gefäßzugangseinrichtung aus einem elektrisch isolierenden Material ausgestaltet oder weist ein solches an entsprechender Stelle auf, so bietet die vorliegende Erfindung einen weiteren Vorteil dahingehend, dass eine Überwachung - anders als bei Verfahren des Standes der Technik, bei welchen die Integrität eines Stromkreises unter Einbeziehung einer elektrisch leitfähigen Kanüle zur Erkennung einer Dekonnektion eingesetzt wird - noch immer funktioniert.

Auch ein sehr schnelles Herausreißen einer Nadel aus einem Gefäß wird sicher erkannt. Hierin unterscheidet sich die erfindungsgemäße Vorrichtung vorteilhaft von Systemen, welche auf dem Erkennen von Feuchte aufgrund von Blutaustritt basieren. Denn beim raschen Herausreißen, beispielsweise beim Umdrehen des Patienten im Bett, kann es sein, dass der Freistrahl gar nicht mehr auf den an der Nadel befestigten Feuchtesenor auftrifft. Eine Dämpfung oder ein verändertes Schwingungsverfahren wird hingegen auch in einem solchen Fall erkannt.

Die erfindungsgemäße Vorrichtung kann allgemein ausgedrückt vorteilhaft den Dämpfungszustand bzw. die Bedämpfung einer Gefäßzugangseinrichtung, welche beispielsweise eine Kanüle, einen Flügel, einen Schlauch und einen Pflasterstreifen aufweist, messen. Dies kann zum Erkennen einer arteriellen oder venösen Nadeldissektion oder - diskonnektion ebenso wie für zum Erkennen der De- bzw. Diskonnektion einer Infusionsnadel und zu einer Vielzahl weiterer Zwecke vorteilhaft eingesetzt werden.

Mittels der erfindungsgemäßen Vorrichtung ist somit vorteilhaft möglich, bereits eine drohende Dekonnektion, z. B. wegen Lösen der Befestigung der Gefäßzugangseinrichtung am Patienten, Lageänderung der Gefäßzugangseinrichtung, zu erkennen.

Die vorliegende Erfindung wird im Folgenden anhand bevorzugter Ausführungsformen unter Bezugnahme auf die angehängte Zeichnung beschrieben. Dabei werden gleiche Bezugszeichen zum Bezeichnen gleicher Elemente in den Figuren verwendet. In der Zeichnung gilt:
- Fig. 1: zeigt schematisch vereinfacht eine mögliche Anordnung der erfindungsgemäßen Vorrichtung;
- Fig. 2: zeigt schematisch vereinfacht eine Ausführungsform der erfindungsgemäßen Vorrichtung in vergrößerter Darstellung;
- Fig. 3: zeigt vergrößert eine weitere Ausführungsform der erfindungsgemäßen Vorrichtung; und
- Fig. 4: zeigt schematische Graphen bei der Überwachung einer Gefäßzugangseinrichtung mittels der erfindungsgemäßen Vorrichtung.

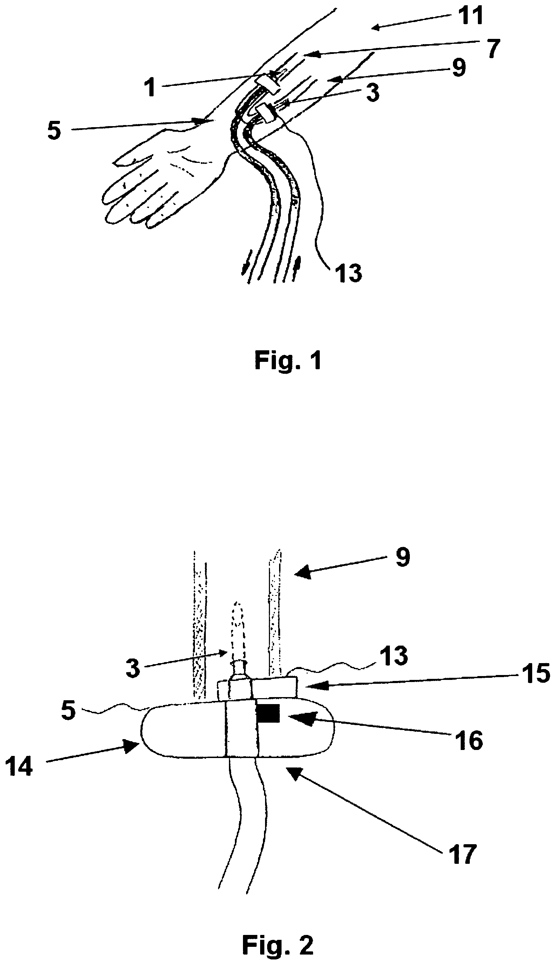

Fig. 1 zeigt eine arterielle Konnektionsnadel 1 und eine venöse Konnektionsnadel 3, welche als Beispiele von Gefäßzugangseinrichtungen mit einem Shunt 5 zwischen einer Arterie 7 und einer Vene 9 des Gefäßsystems eines Patienten 11 verbunden sind. Eine erfindungsgemäße Vorrichtung 13 ist an der venösen Konnektionsnadel 3 befestigt.

Fig. 2 zeigt in einer vergrößerten Darstellung eine erfindungsgemäße Vorrichtung 13, in der ein Signalgeber-/ Signalempfänger 15 und eine Auswerteeinrichtung 16 an einem Clip 17 befestigt sind und dieser mit der venösen Konnektionsnadel 3 verbunden ist. Ferner ist eine mit einem so genannten Punktions- oder Butterflyflügel 14 versehene Gefäßzugangseinrichtung 5 dargestellt. Der Signalgeber-/ Signalempfänger 15 ist hier als eine gemeinsame Einrichtung mit den Funktionen "Senden" und "Empfangen" dargestellt. Die bei Funktionen können jedoch auch mittels zwei getrennter Einrichtungen umgesetzt werden.

Fig. 3 zeigt in vergrößerter Darstellung die erfindungsgemäße Vorrichtung 13, bei welcher der Signalgeber/Signalempfänger 15 einen elektrodynamischen Wandler 19 aufweist. Es können beispielsweise sowohl die von einem Signalgeber aufgenommenen Energiemengen als auch die von einem Signalempfänger aufgenommenen Schwingungssignale aufgezeichnet werden. Vom Signalgeber/Signalempfänger 15 aufgenommene Schallsignale können in elektrische Signale konvertiert und von einer Auswerteeinrichtung ausgewertet werden.

Fig. 4 zeigt schematische Graphen, die die Überwachung einer Gefäßzugangseinrichtung mittels der vorliegenden Vorrichtung darstellen. Die Graphen geben keine realen Messungen wieder, sondern stellen die Messergebnisse schematisch dar. In der linken Spalte der Fig. 4 sind die vom Signalempfänger 15 ausgesandten elektronischen Signale als Spannung U pro Zeiteinheit t und/oder als Frequenz f pro Zeiteinheit t dargestellt. Die rechte Spalte zeigt die von einer Auswerteeinrichtung der erfindungsgemäßen Vorrichtung empfangenen Signale. Die Graphen gehören zeilenweise zusammen, d. h. dass der linke Graph jeweils das ausgesandte Signal (Eingangssignal) und der rechte Graph jeweils das entsprechende empfangene Signal (Ausgangssignal) zeigt. Die strichliniert dargestellten Graphen zeigen jeweils Signale einer herausrutschenden und/oder nicht mehr optimal fixierten Nadel.

Zeile 1 der Figur 4 veranschaulicht die Verwendung eines Senderempfängers, beispielsweise eines elektrodynamischen Wandlers. Das Eingangssignal ist ein Sprungsignal, das Ausgangssignal ist die zugehörige Sprungantwort.

Zeile 2 der Figur 4 veranschaulicht die Verwendung von diskretem Sender und Empfänger, beispielsweise eines elektrodynamischen Wandlers. Das Eingangssignal ist ein Impuls, das Ausgangssignal ist die zugehörige Impulsantwort. Zeile 3 der Figur 4 veranschaulicht die Verwendung eines Senderempfängers, beispielsweise eines elektrodynamischen Wandlers. Der linke Graph zeigt als Eingangssignal einen so genannten "Frequency Sweep". Der rechte Graph zeigt das zugehörige Ausgangssignal als Funktion des aufgenommenen Stroms bei einem Sweep mit konstanter Spannungsamplitude am Senderempfänger als "auszuwertendes Signal". Das strichlinierte Signal 21 veranschaulicht das Herausrutschen einer Konnektionsnadel 1, 3. Das mit durchgezogener Linie gezeichnete Signal 23 veranschaulicht eine korrekte Konnektierung.

Die vorliegende Erfindung ist nicht auf die vorstehend beschriebenen Ausführungsformen beschränkt, diese dienen lediglich der Veranschaulichung.

## Patentansprüche

1. Vorrichtung (13) zum Überwachen einer
Gefäßzugangseinrichtung (5), mit
wenigstens einem Signalgeber (15) zum Erzeugen von Schwingungen zum Anregen eines Abschnitts der Gefäßzugangseinrichtung (5) zum Schwingen, wobei der Abschnitt der Gefäßzugangseinrichtung (5) dem Herstellen einer Verbindung zwischen der Gefäßzugangseinrichtung (5) und dem Gefäßsystem des Patienten dient und wenigstens zum Teil in das Gefäßsystem einführbar ist;
wenigstens einem Signalempfänger (15) zum Erfassen von Schwingungen des Abschnitts der Gefäßzugangseinrichtung (5) ;
und mit wenigstens einer Auswerteeinrichtung (16) zum Auswerten der erfassten Schwingungen.

2. Vorrichtung (13) nach Anspruch 1, wobei der Signalgeber (15) und der Signalempfänger (15) in einer Struktur miteinander verbunden vorliegen.

3. Vorrichtung (13) nach Anspruch 1 oder 2, wobei der Signalgeber (15) und der Signalempfänger (15) körperlich miteinander verbunden sind.

4. Vorrichtung (13) nach einem der vorangegangenen Ansprüche, wobei der Signalgeber (15) und der Signalempfänger (15) vorgesehen und geeignet sind an einem Abschnitt (1, 3) an der Gefäßzugangseinrichtung (5) angeordnet zu werden.

5. Vorrichtung (13) nach einem der vorangegangenen Ansprüche, mit einer Befestigungseinrichtung (17) zum Befestigen der Vorrichtung (13) an der Gefäßzugangseinrichtung (5) oder einem Abschnitt hiervon.

6. Vorrichtung (13) nach einem der vorangegangenen Ansprüche, wobei die Auswerteeinrichtung (16) zum Auswerten eines Schwingungsverhaltens und/oder einer Dämpfung und/oder eines Ausschwingverhaltens der Gefäßzugangseinrichtung (5) ausgelegt ist.

7. Vorrichtung (13) nach Anspruch 6, wobei die Auswerteeinrichtung (16) vorbereitet und geeignet ist, die vom Signalempfänger (15) empfangenen Signale oder Energie auszuwerten.

8. Vorrichtung (13) nach Anspruch 6 oder 7, wobei die Auswerteeinrichtung (16) vorbereitet und geeignet ist, die vom Signalsender (15) aufgenommene Energie auszuwerten.

9. Vorrichtung (13) nach einem der vorangegangenen Ansprüche, mit einer Einrichtung (16) zum Ermitteln der Dämpfung der ausgesendeten Schwingungssignale oder des Schwingungsverhaltens.

10. Vorrichtung (13) nach Anspruch 9, mit einer Einrichtung (16) zum Vergleichen der ermittelten Dämpfung mit bekannten Dämpfungen.

11. Vorrichtung (13) nach Anspruch 9, mit einer Einrichtung (16) zum Vergleichen des ermittelten Schwingungsverhaltens mit bekannten Schwingungsverhalten.

12. Vorrichtung (13) nach einem der vorangegangenen Ansprüche, mit einer Kommunikationseinrichtung zum Verbinden des Signalgebers (15) und/oder des Signalempfängers (15) mit der Auswerteeinrichtung (16) und/oder mit einer Behandlungsvorrichtung.

13. Vorrichtung (13) nach einem der vorangegangenen Ansprüche, mit einer Steuerungs- oder Regelungseinrichtung zum Beeinflussen der Zuführung eines Mediums über die Gefäßzugangseinrichtung (5).

14. Vorrichtung (13) nach einem der vorangegangenen Ansprüche mit einer Gefäßzugangseinrichtung (5).

15. Vorrichtung (13) nach Anspruch 14, wobei die Gefäßzugangseinrichtung (5) ein elektrisch isolierendes Material aufweist oder aus diesem besteht.

## Claims

1. An apparatus (13) for monitoring a vascular access device (5), comprising:
- at least one signals emitter (15) for generating vibrations to induce a portion of the vascular access device (5) to vibrate, wherein the portion of the vascular access device (5) serves to establish a connection between the vascular access device (5) and the vascular system of the patient and can be at least partially inserted into the vascular system;
- at least one signals receiver (15) for detecting vibrations of the portion of the vascular access device (5);
- and at least an evaluation device (16) for evaluating the detected vibrations.

2. The apparatus (13) according to claim 1, wherein the signals emitter (15) and the signals receiver (15) are present, connected with one another, in a structure.

3. The apparatus (13) according to claim 1 or 2, wherein the signals emitter (15) and the signals receiver (15) are physically connected with each another.

4. The apparatus (13) according to anyone of the preceding claims, wherein the signals emitter (15) and the signals receiver (15) are provided and adapted to be arranged at a portion (1, 3) of the vascular access device (5).

5. The apparatus (13) according to anyone of the preceding claims, comprising a fastening device (17) for fastening the apparatus (13) to the vascular access device (5) or to a portion thereof.

6. The apparatus (13) according to anyone of the preceding claims, wherein the evaluation device (16) is designed for evaluating a vibration behaviour and/or a damping and/or a swing-out behaviour of the vascular access device (5).

7. The apparatus (13) according to claim 6, wherein the evaluation device (16) is prepared and adapted for evaluating the signals or energy received by the signals receiver (15) .

8. The apparatus (13) according to claim 6 or 7, wherein the evaluation device (16) is prepared and adapted for evaluating the energy absorbed by the signals transmitter (15).

9. The apparatus (13) according to anyone of the preceding claims, comprising a device (16) for determining the damping of the emitted vibration signals or the vibration behaviour.

10. The apparatus (13) according to claim 9, comprising a device (16) for comparing the determined damping with known dampings.

11. The apparatus (13) according to claim 9, comprising a device (16) for comparing the determined vibration behaviour with known vibration behaviours.

12. The apparatus (13) according to anyone of the preceding claims, comprising a communication device for connecting the signals emitter (15) and/or the signals receiver (15) with the evaluation device (16) and/or with a treatment apparatus.

13. The apparatus (13) according to anyone of the preceding claims, comprising a control or regulating device for influencing the supply of a medium via the vascular access device (5).

14. The apparatus (13) according to anyone of the preceding claims, comprising a vascular access device (5).

15. The apparatus (13) according to claim 14, wherein the vascular access device (5) comprises or consists of an electrically insulating material.

## Revendications

1. Un appareil (13) pour surveiller un dispositif d'accès vasculaire (5), comprenant:
- au moins un émetteur de signaux (15) permettant de générer des vibrations afin d'inciter une section du dispositif d'accès vasculaire (5) à vibrer, où la section du dispositif d'accès vasculaire (5) sert à établir une liaison entre le dispositif d'accès vasculaire (5) et le système vasculaire du patient et peut être insérée au moins partiellement dans le système vasculaire;
- au moins un récepteur de signaux (15) pour détecter les vibrations de la section du dispositif d'accès vasculaire (5);
- et au moins un dispositif d'évaluation (16) pour évaluer les vibrations détectées.

2. L'appareil (13) selon la première revendication, où l'émetteur de signaux (15) et le récepteur de signaux (15) sont présents, reliés l'un à l'autre, dans une structure.

3. L'appareil (13) selon la revendication 1 ou 2, où l'émetteur de signaux (15) et le récepteur de signaux (15) sont physiquement reliés l'un à l'autre.

4. L'appareil (13) selon l'une quelconque des revendications précédentes, où l'émetteur de signaux (15) et le récepteur de signaux (15) sont prévus et adaptés pour être agencés sur une section (1, 3) du dispositif d'accès vasculaire (5).

5. L'appareil (13) selon l'une quelconque des revendications précédentes, comprenant un dispositif de fixation (17) pour fixer l'appareil (13) au dispositif d'accès vasculaire (5) ou à une section de celui-ci.

6. L'appareil (13) selon l'une quelconque des revendications précédentes, où le dispositif d'évaluation (16) est conçu pour évaluer un comportement vibratoire et/ou une atténuation et/ou un comportement d'atténuation vibratoire du dispositif d'accès vasculaire (5).

7. L'appareil (13) selon la revendication 6, où le dispositif d'évaluation (16) est préparé et adapté pour évaluer les signaux ou l'énergie reçu(s)e par le récepteur de signaux (15).

8. L'appareil (13) selon la revendication 6 ou 7, où le dispositif d'évaluation (16) est préparé et adapté pour évaluer l'énergie absorbée par l'émetteur de signaux (15).

9. L'appareil (13) selon l'une quelconque des revendications précédentes, comprenant un dispositif (16) pour déterminer l'atténuation des signaux de vibration émis ou du comportement vibratoire.

10. L'appareil (13) selon la revendication 9, comprenant un dispositif (16) pour comparer l'atténuation déterminée avec des atténuations connues.

11. L'appareil (13) selon la revendication 9, comprenant un dispositif (16) pour comparer le comportement vibratoire déterminé avec des comportements vibratoires connus.

12. L'appareil (13) selon l'une quelconque des revendications précédentes, comprenant un dispositif de communication pour relier l'émetteur de signaux (15) et/ou le récepteur de signaux (15) au dispositif d'évaluation (16) et/ou à un appareil de traitement.

13. L'appareil (13) selon l'une quelconque des revendications précédentes, comprenant un dispositif de commande ou de régulation pour influencer l'introduction d'un fluide via le dispositif d'accès vasculaire (5).

14. L'appareil (13) selon l'une quelconque des revendications précédentes, comprenant un dispositif d'accès vasculaire (5).

15. L'appareil (13) selon la revendication 14, où le dispositif d'accès vasculaire (5) comprend ou est constitué d'un matériau électriquement isolant.
